(19) Europäisches Patentamt
European Patent Office
Office européen des brevets

(11) Numéro de publication: **0 161 143**
**B1**

# (12) FASCICULE DE BREVET EUROPEEN

(45) Date de publication du fascicule du brevet: **12.04.89**

(51) Int. Cl.⁴: **C 07 D 285/18**, A 61 K 31/54 // C07C161/00

(21) Numéro de dépôt: **85400611.1**

(22) Date de dépôt: **28.03.85**

(54) **Nouvelles 1,2,4-thiadiazines et leurs sels, leur procédé et leurs intermédiaires de préparation, leur application à titre de médicaments et les compositions les renfermant.**

(30) Priorité: **02.04.84 GB 8408447**

(43) Date de publication de la demande: **13.11.85 Bulletin 85/46**

(45) Mention de la délivrance du brevet: **12.04.89 Bulletin 89/15**

(84) Etats contractants désignés: **AT BE CH DE FR IT LI LU NL SE**

(56) Documents cités:
**DE-B-2 530 792**
**FR-A-2 249 673**

**JOURNAL OF ORGANIC CHEMISTRY, vol. 38, no. 1, 12 janvier 1973, pages 20-26, Columbus, Ohio, US; T.R. WILLIAMS et al.: "Stereochemistry of sulfur compounds. IV. New ring systems of carbon, nitrogen, and chiral sulfur"**
**JOURNAL OF ORGANIC CHEMISRY, vol. 42, no. 6, 18 mars 1977, pages 952-958, Columbus, Ohio, US; K. SCHAFFNER-SABBA et al.: "Synthesis and chemistry of cyclic sulfoximines"**

(73) Titulaire: **ROUSSEL-UCLAF, 35, boulevard des Invalides, F-75007 Paris (FR)**

(72) Inventeur: **Jones, Stuart Donald, 20, Bishopsfield, Cricklade Wiltshire (GB)**
Inventeur: **Kennewell, Peter David, 10 St. Helen's View Okus, Swindon Wiltshire (GB)**
Inventeur: **Tully, Wilfred Roger, 5 Saint Peter's Road, Cirencester Gloucestershire (GB)**

(74) Mandataire: **Vieillefosse, Jean-Claude et al, Département des Brevets ROUSSEL UCLAF B.P no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 161 143 B1

ACTORUM AG

## Description

La présente invention concerne de nouvelles 1,2,4-thiadiazines et leurs sels, le procédé et des intermédiaires de préparation, l'application à titre de médicaments de ces nouveaux produits et les compositions les renfermant.

La demande française FR-A 2 249 673 décrit des 1,2,4-benzothiadiazines présentant des propriétés dépressives sur le système nerveux central et des propriétés diurétiques. La demande allemande DE-B 2 530 792 décrit des dérives de 1,2,4-benzothia (IV) diazines présentant des propriétés hypotensives, spasmolytiques, diurétiques et dépressives sur le système nerveux central.

L'invention a pour objet de nouvelles 1,2,4-thiadiazines, caractérisées en ce qu'elles répondent à la formule générale (I):

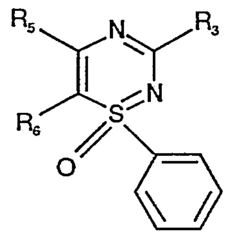

(I)

dans laquelle $R_3$ représente un atome d'hydrogène, un radical alcoyle renfermant de 1 à 6 atomes de carbone, un radical hydroxy, un radical trifluorométhyle, un radical alcoxy renfermant de 1 à 6 atomes de carbone, un radical alcoylthio renfermant de 1 à 6 atomes de carbone, ou $R_3$ représente un groupement amino,

- le terme atome d'halogène désigne par exemple un atome de chlore ou de brome.

Certains produits de formule (I) et notamment les produits pour lesquels $R_3$ représente un radical $-NR_1R_2$ présentent un caractère basique; dans ce cas, il est possible de préparer des sels d'addition avec des acides minéraux ou organiques.

Ces sels peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfoniques, tels que l'acide méthane sulfonique et arylsulfoniques, tels que l'acide benzène sulfonique.

Parmi les produits, objet de l'invention, on peut citer notamment les dérivés répondant à la formule générale (I) ci-dessus, ainsi que leurs sels, caractérisés en ce que dans ladite formule (I), $R_3$ représente un atome d'hydrogène, un radical alcoyle renfermant de 1 à 6 atomes de carbone, un radical alcoylthio renfermant de 1 à 6 atomes de carbone, un radical hydroxy, un radical alcoxy renfermant de 1 à 6 atomes de carbone, un radical trifluorométhyle, un groupement amino, un radical pyrrolidino, pipéridino, morpholino, thiomorpholino, pipérazin-1-yl ou 4-(alcoyl $C_{1-3}$) pipérazin-1-yl, $R_5$ représente un groupement phényle, chlorophényle, méthyl-phényle ou méthoxy phényle, $R_6$ représente un atome d'hydrogène, un atome de brome ou un radical méthyle.

Parmi les produits, objet de l'invention, on retient plus particulièrement les dérivés répondant à la formule (I) ci-dessus, ainsi que leurs sels, caractérisés en ce que dans ladite formule (I), $R_3$ représente un atome d'hydrogène, un radical méthyle, un radical trifluorométhyle, un radical hydroxy, un radical éthoxy, un radical méthylthio ou éthylthio, un groupement amino, un groupement pipéridino ou un groupement 4-(méthyl)-pipérazin-1-yl, $R_5$ représente un groupement phényle, o-chloro-phényle, m-méthyl-phényle ou m-méthoxy-phényle, $R_6$ représente un atome d'hydrogène, un atome de brome ou un radical méthyle.

Parmi ces derniers, on retient tout particulièrement les produits dont les noms suivent:

- le 1,5-diphényl-1H-1,2,4-thiadiazine-1-oxyde, un hétérocycle saturé renfermant de 4 à 8 atomes de carbone, pouvant renfermer un autre atome d'azote, ledit atome d'azote pouvant être substitué par un radical alcoyle renfermant de 1 à 3 atomes de carbone, $R_5$ représente un groupement

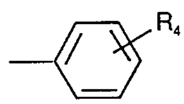

dans lequel $R_4$ représente un atome d'hydrogène, un atome d'halogène, un radical alcoyle renfermant de 1 à 3 atomes de carbone ou un radical alcoxy renfermant de 1 à 3 atomes de carbone, $R_6$ représente un atome d'hydrogène, un atome d'halogène ou un radical alcoyle renfermant de 1 à 3 atomes de carbone, étant entendu que $R_6$ ne représente un atome d'halogène que lorsque $R_3$ représente un atome d'hydrogène, un radical hydroxy, un radical alcoyle, alcoxy ou cycloalcoyle éventuellement substitué par un radical alcoyle, ainsi que, le cas échéant, leurs sels d'addition avec les acides minéraux ou organiques.

Les produits de formule (I) possèdent un atome de soufre asymétrique. Ils peuvent de ce fait se présenter sous forme d'énantiomères ou de mélange d'énantiomères, notamment de mélange racémique.

Dans la formule (I) et dans ce qui suit:

- le terme radical alcoyle renfermant de 1 à 6 atomes de carbone désigne un radical méthyle, éthyle, propyle ou isopropyle;
- le terme hétérocycle saturé renfermant de 4 à 8 atomes de carbone, pouvant renfermer en outre un atome d'oxygène, de soufre ou d'azote désigne par exemple un radical pyrrolidino, pipéridino, morpholino, thiomorpholino, pipérazin-1-yl ou 4-(alcoyl $C_{1-3}$)-pipérazin-1-yl;
- le terme radical alcoxy renfermant de 1 à 6 atomes de carbone désigne par exemple un radical méthoxy, éthoxy, propoxy ou isopropoxy;
- le terme radical alcoylthio renfermant de 1 à 6

atomes de carbone désigne par exemple un radical méthylthio, éthylthio ou propylthio;
- le terme radical cycloalcoyle renfermant de 3 à 6 atomes de carbone éventuellement substitué par un radical alcoyle renfermant de 1 à 6 atomes de carbone désigne, par exemple, un radical cyclopropyle, cyclobutyle, cyclopentyle ou cyclohexyle éventuellement substitué par un radical méthyle, éthyle, propyle ou isopropyle.
- le 1,5-diphényl 6-méthyl-1H-1,2,4-thiadiazine-1-oxyde,
- le 1,5-diphényl 3-méthyl-1H-1,2,4-thiadiazine-1-oxyde,
- le 6-bromo 1,5-diphényl-1H-1,2,4-thiadiazine-1-oxyde,
- le 1,5-diphényl 3-méthoxy-1H-1,2,4-thiadiazine-1-oxyde,
- le (-) 1,5-diphényl-1H-1,2,4-thiadiazine-1-oxyde,
- le (+) 3-méthoxy 1,5-diphényl-1H-1,2,4-thiadiazine-1-oxyde, ainsi que leurs sels d'addition avec les acides.

L'invention a également pour objet un procédé de préparation des produits de formule (I), dans laquelle $R_3$ et $R_5$ ont la signification déjà indiquée et $R_6$ représente un atome d'hydrogène, ou un radical alcoyle renfermant de 1 à 3 atomes de carbone, ainsi que leurs sels, caractérisé en ce que l'on fait réagir un produit de formule (II):

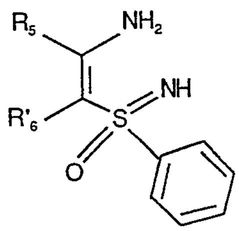

(II)

dans laquelle $R_5$ a la signification déjà indiquée et $R'_6$ représente un atome d'hydrogène ou un radical alcoyle renfermant de 1 à 3 atomes de carbone,
- soit avec un produit de formule (III):

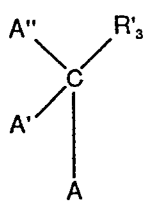

Dans laquelle A, A' et A'', identiques ou différents, représentent un radical éthoxy ou un radical diméthylamino et dans laquelle $R'_3$ représente un atome d'hydrogène ou un radical alcoyle renfermant de 1 à 6 atomes de carbone, pour obtenir un produit de formula ($I_A$):

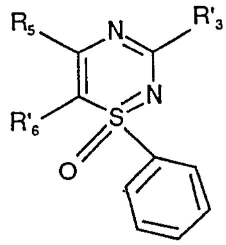

($I_A$)

dans laquelle $R'_3$, $R_5$ et $R'_6$ ont la signification déjà indiquée, que l'on isole et, le cas échéant, on salifie.
- soit avec un clorure ou un anhydride d'acide de formule $R''_3COCl$ ou $(R''_3CO)_2O$, dans laquelle $R''_3$ représente un radical alcoyle renfermant de 1 à 6 atomes de carbone, un radical cycloalcoyle renfermant de 3 à 6 atomes de carbone éventuellement substitué par un radical alcoyle ou un radical trifluorméthyle, pour obtenir un produit de formule ($I_B$):

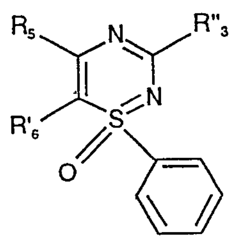

($I_B$)

dans laquelle $R''_3$, $R_5$ et $R'_6$ ont la signification déjà indiquée, que l'on isole, et le cas échéant, on salifie,
- soit, fait réagir le produit de formule (II) avec du N,N'-carbonyl diimidazole de formule ($III_A$):

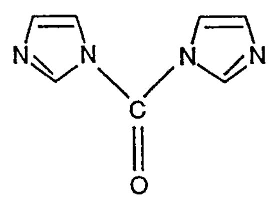

($III_A$)

pour obtenir un produit de formule (IV):

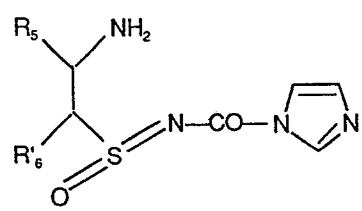

(IV)

dans laquelle $R_5$ et $R'_6$ ont la signification déjà indiquée, que le cas échéant l'on isole, puisque l'on cyclise par chauffage, pour obtenir un produit de formule ($I_C$):

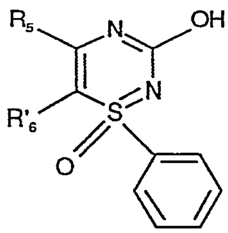

($I_c$)

dans laquelle $R_5$ et $R'_6$ ont la signification déjà indiquée et que, le cas échéant, soit on isole et le cas échéant, salifie le produit de formule ($I_C$) ainsi obtenu, soit on fait réagir ce dernier avec un agent d'halogénation, pour obtenir un produit de formule (V):

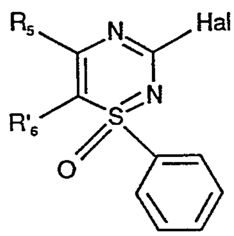

(V)

dans laquelle Hal représente un atome d'halogène, $R_5$ et $R'_6$ ayant la signification déjà indiquée, puis fait réagir ce dernier avec une amine de formule

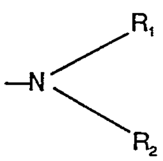

dans laquelle $R_1$ et $R_2$ ont la signification déjà indiquée ou un alcool de formule Alc-O-H ou un alcoylthiol de formule Alc-SH dans laquelle Alc représente un radical alcoyle renfermant de 1 à 6 atomes de carbone, pour obtenir un produit de formule ($I_D$):

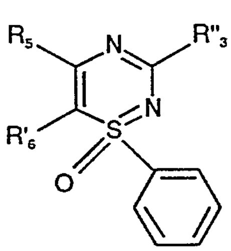

($I_D$)

dans laquelle $R'''_3$ représente un groupement

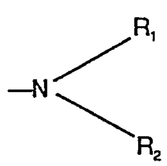

dans lequel $R_1$ et $R_2$ ont la signification déjà indiquée ou $R''_3$ représente un radical alcoxy ou alcoylthio renfermant de 1 à 6 atomes de carbone, $R_5$ et $R'_6$ ayant la signification déjà indiquée, et qu'enfin, l'on isole et si désiré, salifie le produit de formule (I) ainsi obtenu.

Dans des conditions préférentielles de mise en œuvre de l'invention, le procédé de préparation décrit ci-dessus est caractérisé en ce que:

a) la réaction du produit de formule (II) avec le produit de formule (III) est effectuée au sein d'un solvant organique tel que le diméthylformamide à une température comprise entre la température ambiante et la température d'ébullition du mélange réactionnel.

b) La réaction du produit de formule (II) avec le chlorure ou l'anhydride d'acide est effectuée en présence d'une base telle que la triéthylamine, au sein d'un solvant tel que le dichlorométhane.

c) La réaction du produit de formule ($III_A$) est effectuée au sein d'un solvant organique tel que le dichlorométhane.

d) La cyclisation du produit de formule (IV) est effectuée par chauffage du mélange réactionnel, de préférence, à l'ébullition du mélange réactionnel.

e) L'halogénation du produit de formule ($I_C$) est effectuée au moyen d'un agent de bromation ou de chloration tel que l'oxychlorure de phosphore.

f) La réaction du produit de formule (V) avec l'amine de formule

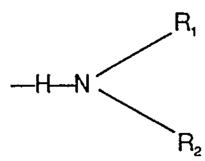

est effectuée au sein d'un alcool de bas poids moléculaire tel que l'éthanol.

g) La réaction du produit de formule (V) avec l'alcool de formule Alc-OH ou avec le thiol de formule Alc-SH est effectuée en présence d'une base qui, dans le cas de l'alcool, est un carbonate alcalin, tel que le carbonate de sodium et, dans le cas du thiol, est un hydrure alcalin tel que l'hydrure de sodium, la réaction étant alors effectuée au sein d'un solvant tel que le diméthylformamide ou le tétrahydrofuranne.

L'invention a aussi pour objet un procédé de préparation des nouvelles 1,2,4-thiadiazines telles que définies par la formule (I) ci-dessus, dans laquelle $R_3$ représente un atome d'hydrogène, un radical hydroxy, un radical alcoyle ou alcoxy renfermant de 1 à 6 atomes de carbone, un radical cycloalcoyle renfermant de 3 à 6 atomes de carbone éventuellement substitué par un radical alcoyle renfermant de 1 à 6 atomes de carbone, $R_5$ a la signification précitée et $R_6$ représente un atome d'halogène, caractérisé en ce que l'on halogène un produit de formule ($I'_A$):

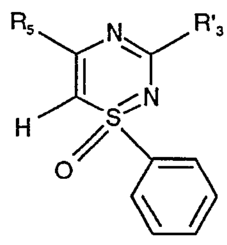

$(I'_A)$

dans laquelle $R'_3$ et $R_5$ ont la signification déjà indiquée, pour obtenir un produit de formule $(I''_A)$:

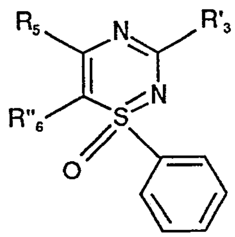

$(I''_A)$

dans laquelle $R'_3$, $R_5$ ont la signification déjà indiquée et $R''_6$ représente un atome d'halogène.

Dans des conditions préférentielles de mise en œuvre de l'invention, le procédé de préparation décrit ci-dessus est caractérisé en ce que l'halogénation est effectuée par réaction de l'halogène considéré, au sein d'un solvant organique tel que le chloroforme.

L'invention a également pour objet une variante du procédé de préparation des produits de formule (I) ci-dessus, dans laquelle $R_3$ représente un radical $-NH_2$ ainsi que de leurs sels, caractérisé en ce que l'on fait réagir un produit de formule (II):

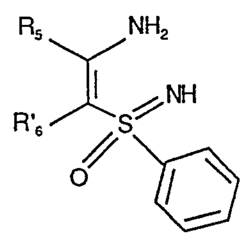

(II)

dans laquelle $R_5$ et $R'_6$ ont la signification déjà indiquée, avec du bromure de cyanogène, en présence d'un agent réducteur, pour obtenir le produit de formule $(I_E)$:

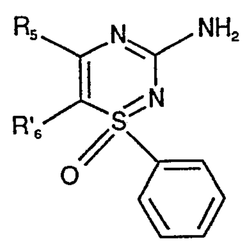

dans laquelle $R_5$ et $R'_6$ ont la signification déjà indiquée que l'on isole et salifie le cas échéant.

Dans des conditions préférentielles de mise en œuvre de l'invention, la variante du procédé de préparation décrite ci-dessus est caractérisée en ce que l'agent réducteur utilisé est l'hydrure de sodium et la réduction est effectuée au sein d'un solvant organique tel que le tétrahydrofurane.

Les sels d'addition avec les acides des produits de formule (I) peuvent, le cas échéant, être préparés en faisant réagir lesdits produits de formule (I) avec un acide tel que ceux décrits précédemment, de préference, en quantité équimoléculaire.

L'invention a également pour objet un procédé de préparation des produits de formule (I) tels que ceux définis précédemment, caractérisé en ce que soit l'on utilise au départ, un produit de formule (II) optiquement actif, soit l'on dédouble les produits de formule (I) obtenus sous forme racémique.

Le dédoublement des produits de formule (I) peut être effectué par les méthodes connues de l'homme de l'art.

Les produits objet de la présente invention, possèdent de très intéressantes propriétés pharmacologiques; ils sont doués notamment de remarquables propriétés anxiolytiques.

Ces propriétés sont illustrées plus loin dans la partie expérimentale. Ces propriétés justifient l'utilisation des nouvelles 1,2,4-thiadiazines de formule (I) ainsi que de leurs sels pharmaceutiquement acceptables, à titre de médicaments.

La présente invention a ainsi également pour objet l'application à titre de médicaments des nouvelles 1,2,4-thiadiazines telles que définies par la formule (I) ainsi que de leurs sels d'addition avec les acides pharmaceutiquement acceptables.

Parmi les médicaments, objet de l'invention, on retient notamment les médicaments caractérisés en ce qu'ils sont constitués par les nouvelles 1,2,4-thiadiazines répondant à la formule (I) dans laquelle $R_3$ représente un atome d'hydrogène, un radical alcoyle renfermant de 1 à 6 atomes de carbone, un radical cycloalcoyle renfermant de 3 à 6 atomes de carbone éventuellement substitué par un radical alcoyle renfermant de 1 à 6 atomes de carbone, un radical alcoylthio renfermant de 1 à 6 atomes de carbone, un radical hydroxy, un radical alcoxy renfermant de 1 à 6 atomes de carbone, un radical trifluorméthyle, un groupement amino, un groupement monoalcoyle ou dialcoylamino dans lesquels le radical alcoyle renferme de 1 à 3 atomes de carbone, un radical pyrrolidino, pipéridino morpholino, thiomorpholino, pipérazin-1-yl ou 4-(alcoyl $C_{1-3}$) pipérazin-1-yl, $R_5$ représente un groupement phényle, chlorphényle, méthyl-phényle ou méthoxy-phényle, $R_6$ représente un atome d'hydrogène, un atome de brome ou un radical méthyle ainsi que par leurs sels d'addition avec les acides pharmaceutiquement acceptables.

Parmi les médicaments, objet de l'invention, on retient plus particulièrement, les médicaments caractérisés en ce qu'ils sont constitués par les nouvelles 1,2,4-thiadiazines répondant à la

formule (I) dans laquelle $R_3$ représente un atome d'hydrogène, un radical méthyle, un radical trifluorométhyle, un radical hydroxy, un radical éthoxy, un radical méthylthio ou éthylthio, un groupement amino, un groupement pipéridino ou un groupement 4-(méthyl)-pipérazin-1-yl, $R_5$ représente un groupement phényle, o-chloro-phényle, m-méthyl-phényle ou m-méthoxy-phényle, $R_6$ représente un atome d'hydrogène, un atome de brome ou un radical méthyle, ainsi que par leurs sels d'addition avec les acides pharmaceutiquement acceptables, et notamment:

- le 1,5-diphényl-1H-1,2,4-thiadiazine-1-oxyde,
- le 1,5-diphényl 6-méthyl-1H-1,2,4-thiadiazine-1-oxyde,
- le 1,5-diphenyl 3-méthyl-1H-1,2,4-thiadiazine-1-oxyde,
- le 6-bromo 1,5-diphényl-1H-1,2,4-thiadiazine-1-oxyde,
- le 1,5-diphényl 3-méthoxy-1H-1,2,4-thiadiazine-1-oxyde,
- le (–) 1,5-diphényl-1H-1,2,4-thiadiazine-1-oxyde,
- le (+) 3-méthoxy 1,5-diphényl-1H-1,2,4-thiadiazine-1-oxyde ainsi que leurs sels d'addition avec les acides pharmaceutiquement acceptables.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être, par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisés en médecine humaine, comme, par exemple, les comprimés, simples ou dragéifiés, les gélules, les capsules, les granulés, les suppositoires, les préparations injectables; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet, à titre de produits industriels nouveaux utiles, notamment pour la préparation des produits de formule (I),

- les produits de formule (V):

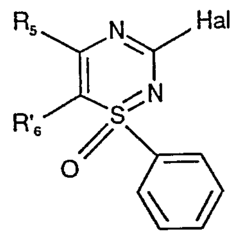

(V)

dans laquelle Hal représente un atome d'halogène, $R_5$ et $R'_6$ ont la signification déjà indiquée.

- Les produits de formule (IV):

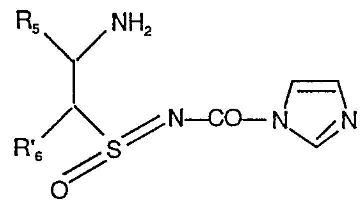

(IV)

dans laquelle $R_5$ et $R'_6$ ont la signification déjà indiquée, sont également nouveaux.

Les produits de formule (II) sont nouveaux. Ils peuvent être préparés par un procédé, caractérisé en ce que l'on fait réagir un aryle nitrile de formule:

$R_5$-CN

dans laquelle $R_5$ a la signification déjà indiquée, avec le dianion d'une alkyl phényl-sulfoximine de formule:

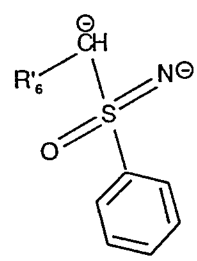

dans laquelle $R'_6$ a la signification déjà indiquée.

Des exemples de préparation des produits de formule (II) sont donnés ci-après dans la partie expérimentale.

Il va être donné maintenant, à titre non limitatif, des exemples de mise en œuvre de l'invention.

Préparation 1:

S-(2-aminostyryl)-S-phenyl-sulfoximine.

On refroidit à 0°C une solution de 8,3 g de méthyl phényl sulfoximine dans 400 cm³ de tétrahydrofuranne puis ajoute une solution 1,4 M de butyllithium dans l'hexane jusqu'à obtention d'une solution claire. On ajoute alors 15 cm³ de benzonitrile, agite 30 minutes, ajoute 100 $cm^3$ d'une solution aqueuse saaturée de chlorure de sodium, sépare la phase organique, la sèche et la concentre à sec.

On triture l'huile brute dans l'éther de pétrole (Eb. 40-60°C) puis chromatographie sur silice (éluant chlorure de méthylène puis chlorure de méthylène - acétate d'éthyle 1-1).

On obtient 8,8 g de produit attendu. F = 95°-96°C après cristallisation dans l'éther.

En opérant comme ci-dessus mais au départ de la sulfoximine et du benzonitrile substitué appropriés, on a préparé les produits des préparations 2 à 5.

Les analyses spectrométriques, les rendements, les points de fusion des produits des préparations 1 à 5 sont données dans le Tableau I ci-après:

Préparation 2
S-[2-amino-2-(4-méthylphényl)-vinyl]S-phényl-sulfoximine.

Préparation 3
S-[2-amino-2-(4-méthoxyphényl)-vinyl]S-phényl-sulfoximine.

Préparation 4
S-[2-amino-2-(2-chlorophényl)-vinyl]S-phényl-sulfoximine.

Préparation 5
S-(2-amino-1-méthylstyryl)-S-phényl-sulfoximine.

Préparation 6
(+) et (-) S-méthyl-S-phényl-sulfoximine.

On a préparé la (+)-S-méthyl-S-phényl sulfoximine selon la méthode décrite par C. Johnson et C. Schroeck (J. Am. Chem. Soc. 1973, 95 (22) 7418-7423; on a obtenu un produit optiquement pur.
$\alpha_D^{20}$ = + 36,7° (c = 4% acétone).

En opérant de la même manière, on a préparé la (-) S-méthyl-S-phényl sulfoximine à partir du produit brut, (renfermant 80 à 85% de (-)S-méthyl-S-phényl sulfoximine) obtenu comme produit secondaire lors de la cristallisation dans l'acétone du sel d'acide 1-10-camphosulfonique de la (+) S-méthyl S-phényl sulfoximine.

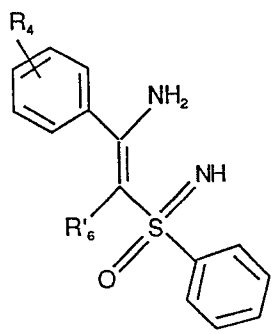

Tableau I

| Préparation | $R'_6$ | R | Rendt % | F °C | Ir. $cm^{-1}$ | RMN ($CDCl_3$ - ppm) |
|---|---|---|---|---|---|---|
| 1 | H | H | 64 | 95-96 | 3420, 3320, 1620, 1550, 1445 | δ3.2 (s,1H,=NH); δ4.95 (s,1H,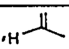); δ6.4 (s,2H,-$NH_2$); δ7.3-8.1 (m,1OH,aryl-H) |
| 2 | H | 4-$CH_3$- | 61 | huile* | 3395, 3290, 1625, 1545, 1515 | δ2.3 (s,3H,aryl$CH_3$); δ2.9 (s,1H,=NH); δ4.9 (s,1H,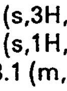); δ6.45 (s,2H,-$NH_2$); δ7-8.1 (m,9H,aryl-H) |
| 3 | H | 4-$CH_3O$- | 68 | huile* | 3420, 3310, 1620, 1545, 1510 | δ3.0 (s,1H,=NH); δ3.8 (s,3H, $OCH_3$); δ4.9 (s,1H,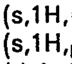); δ6.4 (s,2H,-$NH_2$) δ6.9 (d,J=9,2H,aryl H O - ou $OCH_3$) δ7.4-8 (m,7H aryl H) |
| 4 | H | 2-Cl- | 29 | huile* | 3410, 3320, 1620, 1555, 1450 | δ2.8 (s,1H,NH); δ4.65 (s,1H,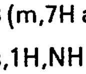); δ6.4 (s,2H,$NH_2$); δ7-8.1 (m,9H,aryl-H) |
| 5 | $CH_3$ | H | 43 | huile* | 3420, 3320, 1615, 1570, 1445 | δ1.6 (s,3H,-$CH_3$); δ3.3 (s,1H=NH); δ6.35 (s,2H,$NH_2$); δ7.3-8.2 (m,10H,aryl H) |

* non cristallisé, utilisé tel quel sous forme d'huile sans purification supplémentaire pour le stade suivant.

Exemple 1

1,5-diphényl-1H-1,2,4-thiadiazine-1-oxyde.

On ajoute 1 g de diméthylformamide diméthyl acétal à une solution comprenant 2 g de S-(2-aminostyryl) S-phényl sulfoximine dans 20 $cm^3$ de diméthylformamide.

On chauffe à 80°C pendant 15 minutes, refroidit, ajoute 100 $cm^3$ d'acétate d'éthyle, lave à l'eau, sèche et concentre à sec.

On recueille une huile qui cristallise dans un mélange éther-éther de pétrole et obtient 1,67 g de produit attendu. F = 110–111 °C. Les résultats de la microanalyse sont donnés dans le Tableau II ci-après.

En utilisant une méthode identique à celle de l'exemple 1 mais en partant du produit correspondant de formule II dans laquelle $R_5$ et $R_6$ ont les significations indiquées dans le Tableau II ci-après, on a obtenu les produits des exemples 2 à 6.

Exemple 2

1-phényl-5-(4-tolyl)-1H-1,2,4-thiadiazine-1-oxyde.

Exemple 3

5-(4-méthoxy phényl) 1-phényl-1H-1,2,4-thiadiazine-1-oxyde,

Exemple 4

5-(2-chlorophenyl) 1-phényl-1H-1,2,4-thiadiazine-1-oxyde,

Exemple 5

1,5-diphényl 6-méthyl 1H-1,2,4-thiadiazine-1-oxyde,

Exemple 6

1,5-diphényl 3-méthyl 1H-1,2,4-thiadiazine-1-oxyde,

Exemple 7

3-hydroxy 1,5-diphényl-1H-1,2,4-thiadiazine-1-oxyde.

On ajoute 16,2 g de N,N'-carbonyldiimidazole à une solution comprenant 12 g de S-(2-aminostyryl)-S-phényl sulfoximine dans 150 $cm^3$ de dichlorméthane et agite pendant 48 heures à température ambiante. On concentre à sec et chromatographie l'huile obtenue sur silice (éluant chloroforme – acétate d'éthyle 9–1). On recueille 15,6 g d'une urée intermédiaire. F = 170–171 °C après cristallisation dans le chloroforme. On chauffe à 200°C, 15,4 g de produit dans 60 $cm^3$ de Dowtherm A pendant 30 minutes, refroidit et dilue par 300 $cm^3$ d'éther. On filtre, lave à l'éther puis au chloroforme, sèche et obtient 12,1 g de produit attendu. F = 304–305°C après recristallisation dans le méthanol.

Exemple 8

3-amino 1,5-diphényl-1H-1,2,4-thiadiazine-1-oxyde.

On ajoute 300 mg d'hydrure de sodium puis 1,06 g de bromure de cyanogène à une solution de 2,58 g de S-(2-aminostyryl)-S-phényl sulfoximine dans 100 $cm^3$ de tétrahydrofuranne et laisse sous agitation pendant 24 heures.

On lave le milieu réactionnel à l'aide d'une solution aqueuse d'hydroxyde de sodium, évapore les solvants, chromatographie l'huile obtenue sur silice (éluant: chlorure de méthylène – acétate d'éthyle 1–1) et obtient 0,58 g de produit attendu. F = 169–170°C après cristallisation dans l'acétate d'éthyle.

Exemple 9

3-amino 1,5-diphényl-1H-1,2,4-thiadiazine-1-oxyde.

Stade A: 3-chloro-1,5-diphényl-1H-1,2,4-thiadiazine-1-oxide.

On chauffe 30 minutes au reflux 13,84 g de produit obtenu à l'exemple 7 dans 50 $cm^3$ d'oxychlorure de phosphore, puis évapore l'excès de solvant sous pression réduite.

On reprend le résidu par du chloroforme, lave à l'eau, sèche et évapore à sec. L'huile obtenue cristallise après trituration dans l'éthanol. On obtient 10,8 g de produit attendu. F = 117–118°C.

Stade B: 3-amino-1,5-diphényl-1H-1,2,4-thiadiazine-1-oxyde.

On ajoute 3 g du produit obtenu au stade A à 75 $cm^3$ d'une solution éthanolique saturée d'ammoniac et chauffe 3 heures à 100°C.

On refroidit le mélange, le verse dans l'eau, filtre le précipité, le lave à l'eau, le sèche et recueille 2,6 g de produit attendu. F= 169–170°C.

En utilisant une méthode analogue à celle du Stade B de l'exemple 9, mais en partant du 3-chloro 1,5-diphéphyl-1H-1,2,4-thiadiazine-1-oxyde de l'amine appropriée de formule:

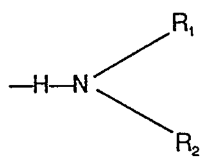

et de l'alcool Alc–OH, on a préparé les composés des exemples 10 à 12.

Exemple 10

1,5-diphényl-3-(N-piperidino)-1H-1,2,4-thiadiazine-1-oxyde.

Exemple 11

3-[1-(4-méthyl pipérazinyl]1,5-diphényl-1H-1,2,4-thiadiazine-1-oxyde.

Exemple 12

3-éthoxy 1,5-diphényl 1H-1,2,4-thiadiazine-1-oxyde.

Exemple 13

6-bromo 1,5-diphényl-1H-1,2,4-thiadiazine-1-oxyde.

On ajoute lentement en 20 minutes, une solution de 2,8 g de brome dans 25 $cm^3$ de chloroform à une solution comprenant 4,5 g de produit obtenu à l'exemple 1 dans 225 $cm^3$ de chloroform et maintient ensuite 30 minutes sous agitation. On concentre à sec, chromatographie l'huile résiduelle sur silice (éluant éther de pétrole - chloroform 1-1) et obtient 4,3 g de produit attendu. F = 135-136 °C après cristallisation dans l'acétate d'éthyle puis dans l'éther.

Exemple 14

1,5-diphényl 3-méthoxy-1H-1,2,4-thiadiazine-1-oxyde.

On opère comme à l'exemple 9 mais en utilisant le méthanol et le carbonate de sodium.

Exemple 15

1,5-diphényl 3-méthylthio-1H-1,2,4-thiadiazine-1-oxyde.

On fait barboter du méthyl mercaptan dans une suspension de 1 g d'hydrure de sodium dans du diméthylformamide jusqu'à obtention d'une solution claire.

On ajoute alors une solution de 3 g de 3-chloro-1,5-diphényl-1H-1,2,4-thiadiazine-1-oxyde préparé comme à l'exemple 9 stade A dans du diméthylformamide et agite une heure à température ambiante, puis dilue par de l'acétate d'éthyle, lave à l'eau et évapore les solvants. On cristallise l'huile obtenue dans le dichlorométhane et dans l'éther et obtient 2,32 g de produit attendu. F = 148-150 °C.

Exemple 16

1,5-diphényl-3-éthylthio-1H-1,2,4-thiadiazine-1-oxyde.

On opère comme à l'exemple 15 mais en utilisant le tétrahydrofuranne comme solvant et l'éthyl mercaptan comme réactif.

Exemple 17

1,5-diphényl-3-trifluorométhyl 1H, 1,2,4-thiadiazine-1-oxyde.

On ajoute 1,33 g de triéthylamine et 2,77 g d'anhydride trifluoroacétique à une solution de 3 g de S-(2-aminostyryl)-S-phényl-sulfoximine dans 100 $cm^3$ de dichlorométhane et agite 2 heures à température ambiante.

On concentre à sec sous pression réduite, reprend le résidu huileux par 100 $cm^3$ de toluène, ajoute 0,4 g de 1,5-diazabicyclo(4,3,0)non-5-ène et chauffe 2 heures au reflux. Après évaporation des solvants sous pression réduite et chromatographie sur silice (éluant éther de pétrole-dichlorométhane), on obtient 1,6 g de produit attendu. F = 136-137 °C après cristallisation dans un mélange éther - éther de pétrole).

Exemple 18

(-)1,5-diphényl-1H-1,2,4-thiadiazine-1-oxyde.

En utilisant une méthode analogue à celle décrite pour la préparation du S-(2-aminostyryl)-S-phényl sulfoximine (préparation 1) et du 1,5-diphényl-1H-1,2,4-thiadiazine-1-oxyde (exemple 1) mais au départ de 5,5 g de (+)-S-méthyl-S-phényl-sulfoximine, on obtient 3,54 g de (-) 1,5-diphényl-1H-1,2,4-thiadiazine-1-oxyde. F = 102-103 °C après cristallisation dans l'éther. $\alpha_D^{21}$ = −120,7° (c = 4,1% acétone).

Exemple 19

(+)1,5-diphényl-1H-1,2,4-thiadiazine-1-oxyde.

En opérant comme à l'exemple 18, au départ de la (-)-S-méthyl-S-phényl-sulfoximine, on obtient le (+)1,5-diphényl-1H-1,2,4-thiadiazine-1-oxyde. $\alpha_D^{21}$ = + 119,4° (c = 4,1% acétone).

Les rendements, points de fusion, résultats de la microanalyse et des analyses spectrométriques des produits des exemples 1 à 19 sont notés dans le Tableau II ci-après.

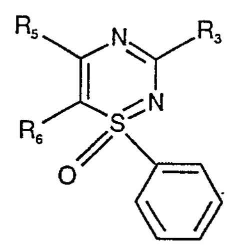

(I)

Tableau II

| Exemple | $R_3$ | $R_5$ | $R_6$ | Rendt. % | F °C | Formule | P.Mol. |
|---|---|---|---|---|---|---|---|
| 1 | -H | Phenyl | -H | 80 | 110-111 | $C_{15}H_{12}N_2OS$ | 268.32 |
| 2 | -H | 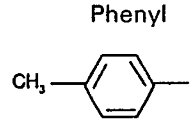 | -H | 74 | 103-103.5 | $C_{16}H_{14}N_2OS$ | 282.35 |
| 3 | -H | 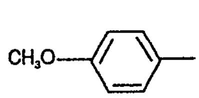 | -H | 57 | 102-104 | $C_{16}H_{14}N_2O_2S$ | 298.34 |

Tableau II (Suite)

| Exemple | $R_3$ | $R_5$ | $R_6$ | Rendt. % | F °C | Formule | P.Mol. |
|---|---|---|---|---|---|---|---|
| 4 | -H | 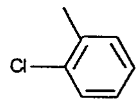 | -H | 79 | 85-87 | $C_{15}H_{11}ClN_2OS$ | 302.76 |
| 5 | -H | Phenyl | $-CH_3$ | 75 | 105-106 | $C_{16}H_{14}N_2OS$ | 282.35 |
| 6 | $-CH_3$ | Phenyl | -H | 86 | 103-104 | $C_{16}H_{14}N_2OS$ | 282.35 |
| 7 | -OH | Pheynl | -H | 84 | 304-305 | $C_{15}H_{12}N_2O_2S$ | 284.32 |
| 8 | $-NH_2$ | Phenyl | -H | 21 | 169-170 | $C_{15}H_{13}N_3OS$ | 283.34 |
| 9 | $-NH_2$ | Phenyl | -H | 93 | 169-170 | $C_{15}H_{13}N_3OS$ | 283.34 |
| 10 | 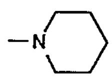 | Phenyl | -H | 61 | 164-165 | $C_{20}H_{21}N_3OS$ | 351.47 |
| 11 | 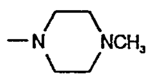 | Phenyl | -H | 50 | 193-194 | $C_{20}H_{22}N_4OS$<br>$0{,}25H_2O$ | 370.99 |
| 12 | -OEt | Phenyl | -H | 74 | 105-107 | $C_{17}H_{16}N_2O_2S$ | 342.39 |
| 13 | -H | Phenyl | -Br | 72 | 135-136 | $C_{15}H_{11}BrN_2OS$ | 347.24 |
| 14 | $OCH_3$ | Phenyl | H | 72 | 99-101 | $C_{16}H_{14}N_2O_2S$ | 298.36 |
| 15 | $SCH_3$ | Phenyl | H | 74 | 148-150 | $C_{16}H_{14}N_2OS_2$<br>$0{,}25H_2O$ | 314.43 |
| 16 | SEt | Phenyl | H | 66 | 78-79 | $C_{17}H_{16}N_2OS_2$ | 328.44 |
| 17 | $-CF_3$ | Phenyl | H | 70 | 136-137 | $C_{16}H_{11}N_2OSF_3$ | 336.32 |
| 18 | H | Phenyl | H | 37+ | 102-103 | $C_{15}H_{12}N_2OS$ | 268.32 |
| 19 | H | Phenyl | H | 52+ | 101-103 | $C_{15}H_{12}N_2OS$ | 268.32 |
| 20 | $-OCH_3$ | Phenyl | H | 20 | 134-135 | $C_{16}H_{14}N_2O_2S$ | 298.36 |

+ Rendement à partir de la S-methyl-S-phényl-sulfoximine optiquement active.

Tableau II (Suite)

| Exemple | Analyse calcule/trouvé | | | | Autres substituants | IR $cm^{-1}$ | RMN* (ppm) |
|---|---|---|---|---|---|---|---|
| | C | H | N | S | | | |
| 1 | 67.14<br>67.22 | 4.51<br>4.63 | 10.44<br>10.34 | 11.95<br>11.73 | -<br>- | 3070, 1590, 1540, 1500 | δ6.2 (d,J=1.5,1H); δ7.2-8.0 (m,10H); δ8.3 (d,J=1.5,1H) |
| 2 | 68.06<br>67.81 | 5.00<br>5.05 | 9.92<br>9.93 | 11.36<br>11.25 | -<br>- | 3030, 1625, 1545, 1515 | δ2.38 (s,3H); δ6.2 (d,J=1.5,1H); δ7.1-8.0 (m,10H); δ8.25 (d,J=1.5,1H) |
| 3 | 64.41<br>64.35 | 4.73<br>4.79 | 9.39<br>9.31 | 10.75<br>10.66 | -<br>- | 3060, 1605, 1540, 1510 | δ3.8 (s,3H); δ6.15 (d,J=1H); δ6.9 (d,J=10,2H), δ7.2-8.0 (m,7H); δ8.2(d,J=1.0,1H) |
| 4 | 59.56<br>59.61 | 3.66<br>3.74 | 9.25<br>9.25 | 10.56<br>10.66 | Cl.11.71<br>11.70 | 3072, 1595, 1560, 1540 | δ6.15 (d,J=2.0,1H); δ7.1-8.0 (m,9H); δ8.12 (d,J=2.0,1H) |
| 5 | 68.06<br>67.78 | 5.00<br>5.02 | 9.92<br>9.77 | 11.36<br>11.33 | -<br>- | 3065, 1580, 1555 | δ1.9 (s,3H); δ7.2-8.0 (m,10H); δ8.07 (s,1H) |
| 6 | 68.06<br>68.18 | 5.00<br>5.10 | 9.92<br>9.86 | 11.36<br>11.23 | -<br>- | 3095, 1580, 1530 | δ2.48 (s,3H); δ6.07 (s,1H); δ7.3-8.0 (m,10H) |
| 7 | 63.34<br>63.30 | 4.26<br>4.31 | 9.86<br>9.88 | 11.28<br>11.24 | -<br>- | 3170, 3060, 2950, 1650, 1600, 1575 | ($D_6$-DMSO) δ6.5 (s,1H); δ7.4-8.1 (m,10H); δ10.82 (s,1H avec $D_2O$) |
| 8 | 63.58<br>63.47 | 4.62<br>4.67 | 14.83<br>14.74 | 11.32<br>11.17 | -<br>- | 3430, 3295, 3100, 1630, 1540 | δ2.45 (s1, approx 0.4H) : δ5.6 (bs) δ5.75 (s) (2.6H combiné); δ7.2-8.0 (m,10H) |

Tableau II (Suite)

| Exemple | Analyse calcule/trouvé C | H | N | S | Autres substituants | IR $cm^{-1}$ | RMN* (ppm) |
|---|---|---|---|---|---|---|---|
| 9 | 63.58<br>63.47 | 4.62<br>4.67 | 14.83<br>14.74 | 11.32<br>11.17 | -<br>- | 3440, 3295, 3100, 1630, 1540 | δ2.45 (s1, approx 0.4H): δ5.6 (bs) et δ5.75 (s) (2.6H combiné); δ7.2–8.0 (m,10H) |
| 10 | 68.34<br>68.00 | 6.02<br>6.13 | 11.96<br>11.98 | 9.12<br>9.02 | -<br>- | 2920, 2842, 1580, 1505 | δ1.6 (s1, 6H); δ3.85 (s1, 4H); δ5.65 (s,1H); δ7.2–8.0 (m,10H) |
| 11 | 64.75<br>64.69 | 6.11<br>6.02 | 15.10<br>15.04 | 8.64<br>8.57 | -<br>- | 3075, 2930, 2680, 1580, 1515 | δ2.28 (s) and δ3.4 (t ou dd, J=6.8, 7H combiné); δ3.9 (t ou dd, J=6.0, 4H); δ5.78 (s,1H); δ7.2–8.1 (m, 10H) |
| 12 | 65.36<br>65.10 | 5.16<br>5.14 | 8.97<br>8.98 | 10.26<br>10.14 | -<br>- | 3040, 1582, 1537 | δ1.4 (t,J=7.0,3H); δ4.45(q,J=7.0, 2H); δ6.02 (s,1H); δ7.3–8.0 (m,10H) |
| 13 | 51.89<br>51.61 | 3.19<br>3.27 | 8.07<br>8.13 | 9.24<br>9.13 | Br.23.01<br>23.08 | 1600, 1580, 1535 | δ7.1–8.1 (m,10H); δ8.27 (s,1H) |
| 14 | 64.41<br>64.31 | 4.73<br>4.77 | 9.39<br>9.32 | 10.75<br>10.61 | -<br>- | 1530, 1470, 1383, 1323, 1238 | δ4.05 (s,3H); δ6.08 (s,1H); δ7.35–8.0 (m,10H) |
| 15 | 60.30<br>60.57 | 4.51<br>4.52 | 8.79<br>8.79 | 20.10<br>19.79 | -<br>- | 1440, 1388, 1370, 1225, 1190 | δ2.6 (s,3H); δ6.05 (s,1H); δ7.3–8.0 (m,10H) |
| 16 | 62.17<br>62.26 | 4.91<br>4.94 | 8.53<br>8.55 | 19.52<br>19.50 | -<br>- | 1445, 1418, 1360, 1220, 1195 | δ1.4 (t,3H); δ3.18 (q,2H); δ6.05 (s,1H); δ7.35–8.0 (m,10H) |
| 17 | 57.14<br>57.14 | 3.30<br>3.38 | 8.33<br>8.26 | 9.53<br>9.56 | F.16.95<br>16.89 | 1540, 1340, 1205, 1190, 1130 | δ6.38 (s,1H); δ7.3–8.1 (m,10H) |
| 18 | 67.14<br>67.13 | 4.51<br>4.59 | 10.44<br>10.42 | 11.95<br>11.85 | -<br>- | 1525, 1445, 1400, 1230, 1190 | δ6.25 (d,J=1.5Hz); δ7.3–8.0 (m,10H); δ8.28 (d,J=1.5Hz) |
| 19 | 67.14<br>67.20 | 4.51<br>4.60 | 10.44<br>10.45 | 11.95<br>11.94 | -<br>- | 1522, 1446, 1397, 1225, 1190 | δ6.25 (d,J=1.5Hz); δ7.3–8.0 (m,10H); δ8.28 (d,J=1.5Hz) |
| 20 | 64.41<br>64.24 | 4.73<br>4.76 | 9.39<br>9.36 | 10.75<br>10.62 | -<br>- | 1530, 1472, 1387, 1325, 1240 | δ4.05 (s,3H); δ6.08 (s,1H); δ7.35–8.0 (M,10H) |

* Jen Hz; solvant CDV13, sauf ceux indiqués.

Exemple 20

(+) 3-méthoxy 1,5-diphényl-1H 1,2,4-thiadiazine-1-oxyde.

En utilisant une méthode analogue à celle décrite pour la préparation de la S-(2-aminostyryl)S-phényl sulfoximine (préparation 1) du 3-hydroxy 1,5-diphényl-1H-1,2,4-thiadiazine-1-oxyde (exemple 7) du 3-chloro-1,5-diphényl-1H-1,2,4-thiadiazine-1-oxyde (exemple 9, stade A) et du 3-méthoxy 1,5-diphényl-1H-1,2,4-thiadiazine-1-oxyde (exemple 14) mais au départ de 8,5 g de la (+) S-méthyl S-phényl sulfoximine, on a obtenu 3,2 g de (+) 3-méthoxy 1,5-diphényl-1H-1,2,4-thiadiazine-1-oxyde. F = 134–135°C après cristallisation dans l'éther.

$[\alpha]_D$ + 123,9° (c = 2% acétone).

Exemple 21

On a préparé des comprimés correspondant à la formulation suivante:

- composé de l'exemple 1 25 mg.
- excipient q.s. pour un comprimé terminé à 150 mg.

(Détail de l'excipient: lactose, talc, amidon, stéarate de magnésium).

Exemple 22

On a préparé des comprimés correspondant à la formulation suivante:

- composé de l'exemple 5 25 mg.
- excipient q.s. pour un comprimé terminé à 150 mg.

(Détail de l'excipient: lactose, talc, amidon, stéarate de magnésium).

Exemple 23

On a préparé des comprimés correspondant à la formulation suivante:

- composé de l'exemple 19 10 mg.

- excipient q.s. pour un comprimé terminé à 150 mg.

(Détail de l'excipient: lactose, talc, amidon, stéarate de magnésium).

Activité pharmacologique.

L'étude de l'activité anxiolytique a été effectuée par la méthode du «Conflict Test» selon Geller et Seifter (Psychopharmacologia, 1960, I, 482) modifiée.

Les valeurs données dans le Tableau III sont les doses efficaces minimum auxquelles il est observé un accroissement des chocs par rapport aux témoins (D.E.M. mg/kg po).

Tableau III

| Exemple | DEM mg/kg po |
|---|---|
| 1 | 5 |
| 3 | 50 |
| 5 | 10 |
| 6 | 10 |
| 8 | 50 |
| 9 | 50 |
| 10 | 50 |
| 11 | 50 |
| 12 | 50 |
| 13 | 10 |
| 14 | 5 |
| 16 | 50 |
| 17 | 50 |
| 18 | 2 |
| 19 | 50 |
| 20 | 5 |

**Revendications**

1. Nouvelles 1,2,4-thiadiazines, sous forme d'énantiomères ou de mélanges d'énantiomères, caractérisées en ce qu'elles répondent à la formule générale (I):

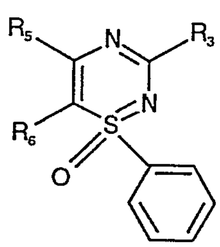

(I)

dans laquelle $R_3$ représente un atome d'hydrogène, un radical alcoyle renfermant de 1 - 6 atomes de carbone, un radical hydroxy, un radical trifluorométhyle, un radical alcoxy renfermant de 1 à 6 atomes de carbone, un radical alcoylthio renfermant de 1 à 6 atomes de carbone, ou $R_3$ représente un groupement amino, un hétérocycle saturé renfermant de 4 à 8 atomes de carbone, pouvant renfermer un autre atome d'azote, ledit atome d'azote pouvant être substitué par un radical alcoyle renfermant de 1 à 3 atomes de carbone, $R_5$ représente un groupement

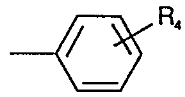

dans lequel $R_4$ représente un atome d'hxdrogène, un atome d'halogène, un radical alcoyle renfermant de 1 à 3 atomes de carbone ou un radical alcoxy renfermant de 1 à 3 atomes de carbone, $R_6$ représente un atome d'hydrogène, un atome d'halogène ou un radical alcoyle renfermant de 1 à 3 atomes de carbone, étant entendu que $R_6$ ne représente un atome d'halogène que lorsque $R_3$ représente un atome d'hydrogène, un radical hydroxy, un radical alcoyle, alcoxy ou cycloalcoyle éventuellement substitué par un radical alcoyle ainsi que, le cas échéant, leurs sels d'addition avec les acides minéraux ou organiques.

2. Dérivés répondant à la formule générale (I) de la revendication 1 ainsi que leurs sels, caractérisés en ce que dans ladite formule (I), $R_3$ représente un atome d'hydrogène, un radical alcoyle renfermant de 1 à 6 atomes de carbone, un radical alcoylthio renfermant de 1 à 6 atomes de carbone, un radical hydroxy, un radical alcoxy renfermant de 1 à 6 atomes de carbone, un radical trifluorméthyle, un groupement amino, un radical pyrrolidino, pipéridino, morpholino, thiomorpholino, pipérazin-1-yl ou 4-(alcoyle $C_{1-3}$) pipérazin-1-yl, $R_5$ représente un groupement phényle, chlorophényle, méthyl-phényle ou méthoxy phényle, $R_6$ représente un atome d'hydrogène, un atome de brome ou un radical méthyle.

3. Dérivés répondant à la formule générale (I) de la revendication 1 ainsi que leurs sels, caractérisés en ce que dans ladite formule (I), $R_3$ représente un atome d'hydrogène, un radical méthyle, un radical trifluorométhyle, un radical hydroxy, radical éthoxy, un radical méthylthio ou éthylthio, un groupement amino, un groupement pipéridino ou un groupement 4-(méthyl)-pipérazin-1-yl, $R_5$ représente un groupement phényle, o-chlorophényle m-méthyl-phényle ou m-méthoxy-phényle, $R_6$ représente un atome d'hydrogène, un atome de brome ou un radical méthyle.

4. L'un quelconque des dérivés de formule (I) telle que définie à la revendication 1, dont les noms suivent:

- le 1,5-diphényl-1H-1,2,4-thiadiazine-1-oxyde,
- le 1,5-diphényl 6-méthyl-1H-1,2,4-thiadiazine-1-oxyde,
- le 1,5-diphényl 3-méthyl-1H-1,2,4-thiadiazine-1-oxyde,
- le 6-bromo 1,5-diphényl-1H-1,2,4-thiadiazine-1-oxyde,
- le 1,5-diphényl 3-méthoxy-1H-1,2,4-thiadiazine-1-oxyde,
- le (-)1,5-diphényl 3-méthoxy-1H-1,2,4-thiadiazine-1-oxyde,
- le (+) 3-méthoxy 1,5-diphényl-1H-1,2,4-thiadiazine-1-oxyde ainsi que leurs sels d'addition avec les acides.

5. Procédé de préparation des nouvelles 1,2,4-thiadiazines telles que définies par la formule (I) de la revendication 1, dans laquelle $R_3$ et $R_5$ ont la signification indiquée à la revendication 1 et $R_6$ représente un atome d'hydrogène ou un radical alcoyle renfermant de 1 à 3 atomes de carbone, ainsi que leurs sels, caractérisé en ce que l'on fait réagir un produit de formule (II):

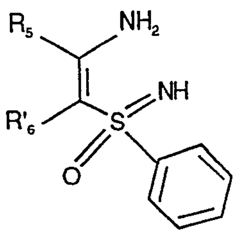

(II)

dans laquelle $R_5$ a la signification déjà indiquée et $R'_6$ représente un atome d'hydrogène ou un radical alcoyle renfermant de 1 à 3 atomes de carbone,

- soit avec un produit de formule (III):

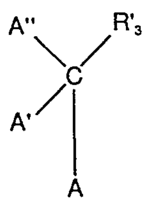

(III)

dans laquelle A, A' et A'', identiques ou différents, représentent un radical éthoxy ou un radical diméthyl amino sans laquelle $R'_3$ représente un atome d'hydrogène ou un radical alcoyle renfermant de 1 à 6 atomes de carbone, pour obtenir un produit de formule ($I_A$):

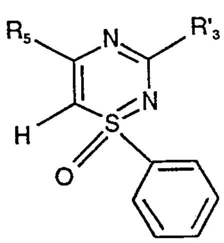

($I'_A$)

dans laquelle $R'_3$, $R_5$ et $R'_6$ ont la signification déjà indiquée, que l'on isole et le cas échéant, on salifie,

- soit avec un chlorure ou un anhydride d'acide de formule $R''_3COCl$ ou $(R''3CO)_2O$ dans laquelle $R''_3$ représente un radical alcoyle renfermant de 1 à 6 atomes de carbone, un radical cycloalcoyle renfermant de 3 à 6 atomes de carbone éventuellement substitué par un radical alcoyle ou un radical trifluorméthyle, pour obtenir un produit de formule ($I_B$):

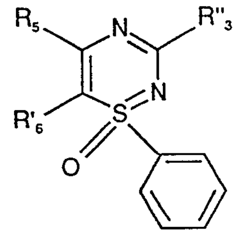

($I_B$)

dans laquelle $R''_3$, $R_5$ et $R'_6$ ont la signification déjà indiquée, que l'on isole et le cas échéant, on salifie,

- soit on fait réagir le produit de formule (II) avec du N,N'-carbonyl diimidazole de formule ($III_A$):

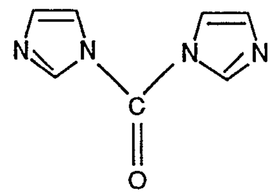

($III_A$)

pour obtenir un produit de formule (IV):

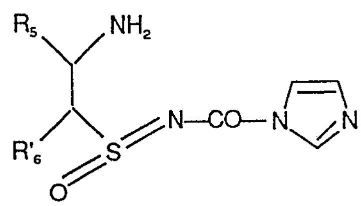

(IV)

dans laquelle $R_5$ et $R'_6$ ont la signification déjà indiquée que le cas échéant l'on isole puis que l'on cyclise par chauffage pour obtenir un produit de formule ($I_C$):

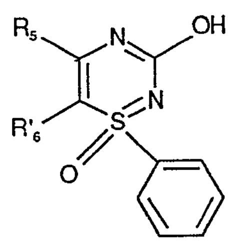

($I_C$)

dans laquelle $R_5$ et $R'_6$ ont la signification déjà indiquée et que le cas échéant, soit on isole et le cas échéant, salifie le produit de formule ($I_C$) ainsi obtenu, soit on fait réagir ce dernier avec un agent d'halogénation, pour obtenir un produit de formule (V):

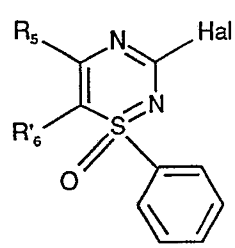

(V)

dans laquelle Hal représente un atome d'halogène, $R_5$ et $R'_6$ ayant la signification déjà indiquée, puis fait réagir ce dernier avec une amine de formule

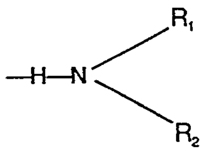

dans laquelle $R_1$ et $R_2$ ont la signification 40 indiquée à la revendication 1 ou un alcool de formule Alc-OH ou un alcoylthiol de formule Alc-SH dans laquelle Alc représente un radical alcoyle renfermant de 1 à 6 atomes de carbone, pour obtenir un produit de formule ($I_D$):

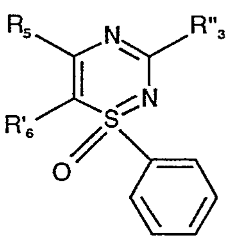

($I_D$)

dans laquelle $R''_3$ représente un groupement

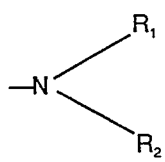

dans lequel $R_1$ et $R_2$ ont la signification déjà indiquée ou $R''_3$ représente un radical alcoxy ou alcoylthio renfermant de 1 à 6 atomes de carbone, $R_5$ et $R'_6$ ayant la signification déjà indiquée et qu'enfin, l'on isole et si désiré, salifie le produit de formule (I) ainsi obtenu.

6. Procédé selon la revendication 5, caractérisé en ce que:

a) la réaction du produit de formule (II) avec le produit de formule (III) est effectuée au sein d'un solvant organique tel que le diméthylformamide à une température comprise entre la température ambiante et la température d'ébullition du mélange réactionnel.

b) la réaction du produit de formule (II) avec l'anhydride trifluoroacétique est effectuée en présence d'une base telle que la triéthylamine, au sein d'un solvant tel que le dichlorométhane;

c) la réaction du produit de formule (II) avec le produit de formule ($III_A$) est effectuée au sein d'un solvant organique tel que le dichlorométhane;

d) la cyclisation du produit de formule (IV) est effectuée par chauffage du mélange réactionnel, de préférence, à l'ébullition du mélange réactionnel;

e) l'halogénation du produit de formule ($I_C$) est effectuée au moyen d'un agent de bromation ou de chloration tel que l'oxychlorure de phosphore;

f) la réaction du produit de formule (V) avec l'amine de formule $H-NR_1R_2$ est effectuée au sein d'un alcool de bas poids moléculaire tel que l'éthanol;

g) la réaction du produit de formule (V) avec l'alcool de formule Alc-OH ou avec le thiol de formule Alc-SH est effectuée en présence d'une base qui, dans le cas de l'alcool est un carbonate alcalin, tel que le carbonate de sodium et dans le cas du thiol est un hydrure alcalin tel que l'hydrure de sodium, la réaction étant alors effectuée au sein d'un solvant tel que le diméthylformamide ou le tétrahydrofuranne.

7. Procédé de préparation des nouvelles 1,2,4-thiadiazines telles que définies par la formule (I) de la revendication 1, dans laquelle $R_3$ représente un atome d'hydrogène, un radical hydroxy, un radical alcoyle ou alcoxy renfermant de 1 à 6 atomes de carbone, un radical cycloalcoyle renfermant de 3 à 6 atomes de carbone, éventuellement substitué par un radical alcoyle renfermant de 1 à 6 atomes de carbone, $R_5$ a la signification donnée à la revendication 1 et $R_6$ représente un atome d'halogène, caractérisé en ce que l'on halogène un produit de formule ($I'_A$):

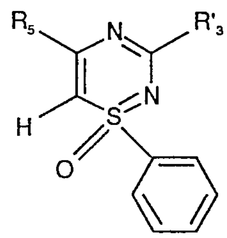

($I'_A$)

dans laquelle $R'_3$ et $R_5$ ont la signification déjà indiquée, pour obtenir un produit de formule ($I''_A$):

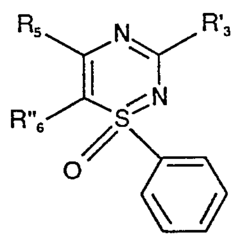

($I''_A$)

dans laquelle $R'_3$, $R_5$ ont la signification déjà inidiquée et $R''_6$ représente un atome d'halogène.

8. Procédé de préparation selon la revendication 7, caractérisé en ce que l'halogénation est effectuée par réaction de l'halogène considéré, au sein d'un solvant organique tel que le chloroforme.

9. Variante du procédé de préparation des produits de formule (I) dans laquelle $R_3$ représente un radical $-NH_2$ ainsi que de leurs sels, caractérisé en ce que l'on fait réagir un produit de formule (II):

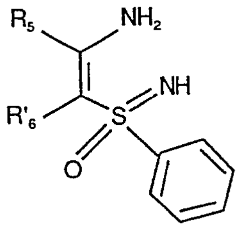

(II)

dans laquelle $R_5$ et $R'_6$ ont la signification indiquée à la revendication 5 avec du bromure de cyanogène, en présence d'un agent réducteur, pour obtenir le produit de formule ($I_E$):

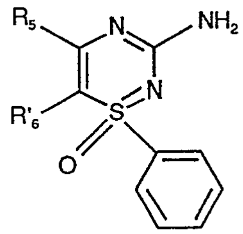

($I_E$)

dans laquelle $R_5$ et $R'_6$ ont la signification déjà indiquée, que l'on isole et salifie le cas échéant.

10. Procédé de préparation selon la revendication 9, caractérisé en ce que l'agent réducteur utilisé est l'hydrure de sodium et la réduction est effectuée au sein d'un solvant organique tel que le tétrahydrofuranne.

11. Procédé selon l'une quelconque des revendications 5, 7 ou 9, caractérisé en ce que soit l'on utilise au départ, un produit optiquement actif, soit l'on dédouble les produits de formule (I) obtenus sous forme racémique et obtient les produits de formule (I) sous leurs formes énantiomères.

12. Médicaments, caractérisés en ce qu'ils sont constitués par les nouvelles 1,2,4-thiadiazines telles que définies par la formule (I) de la revendication 1 ainsi que par leurs sels d'addition avec les acides pharmaceutiquement acceptables.

13. Médicaments, selon la revendication 12, caractérisés en ce qu'ils sont constitués par les 1,2,4-thiadiazines telles que définies à la revendication 2 ou 3 ainsi que par leurs sels d'addition avec les acides pharmaceutiquement acceptables.

14. Médicaments, selon la revendication 12, caractérisés en ce qu'ils sont constitués par les dérivés tels que définis à la revendication 4 ainsi que par leurs sels d'addition avec les acides pharmaceutiquement acceptables.

15. Compositions pharmaceutiques, caractérisées en ce qu'elles renferment à titre de principe actif, l'un au moins des médicaments tels que définis à l'une quelconque des revendications 12, 13 ou 14.

16. A titre de produits industriels nouveaux, les produits de formule (V):

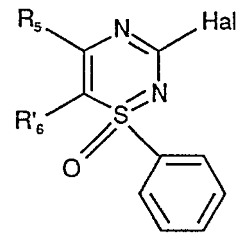

(V)

dans laquelle Hal représente un atome d'halogène, $R_5$ et $R'_6$ ont la signification déjà indiquée.

**Patentansprüche**

1. Neue 1,2,4-Thiadiazine in Form der Enantiomeren oder der Enantiomeren-Gemische, dadurch gekennzeichnet, dass sie der allgemeinen Formel (I) entsprechen:

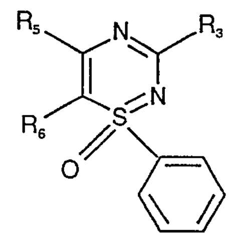

(I)

worin $R_3$ ein Wasserstoffatom, einen Alkylrest mit 1 bis 6 Kohlenstoffatomen, eine Hydroxygruppe, einen Trifluormethylrest, einen Alkoxyrest mit 1 bis 6 Kohlenstoffatomen, einen Alkylthiorest mit 1 bis 6 Kohlenstoffatomen darstellt oder $R_3$ für eine Aminogruppe, einen gesättigten Heterocyclus mit 4 bis 8 Kohlenstoffatomen, der ein weiteres Stickstoffatom enthalten kann, steht, wobei das Stickstoffatom durch einen Alkylrest mit 1 bis 3 Kohlenstoffatomen substituiert sein kann, $R_5$ eine Gruppe darstellt, worin $R_4$ ein Wasserstoffatom, ein

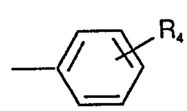

Halogenatom, einen Alkylrest mit 1 bis 3 Kohlenstoffatomen oder einen Alkoxyrest mit 1 bis 3 Kohlenstoffatomen bedeutet, $R_6$ für ein Wasserstoffatom, ein Halogenatom oder einen Alkylrest mit 1 bis 3 Kohlenstoffatomen steht, wobei $R_6$ kein Halogenatom darstellen kann, wenn $R_3$ für ein Wasserstoffatom, eine Hydroxygruppe, einen Alkylrest, einen Alkoxyrest oder einen gegebenenfalls durch einen Alkylrest substituierten Cycloalkylrest steht, sowie gegebenenfalls deren Additionssalze mit Mineral- oder organischen Säuren.

2. Derivate der allgemeinen Formel (I) gemäss Anspruch 1 sowie deren Salze, dadurch gekennzeichnet, dass in der Formel (I) $R_3$ für ein Wasserstoffatom, einen Alkylrest mit 1 bis 6 Kohlenstoffatomen, einen Alkylthiorest mit 1 bis 6 Kohlenstoffatomen, eine Hydroxygruppe, eine Alkoxygruppe mit 1 bis 6 Kohlenstoffatomen, eine Trifluormethylgruppe, eine Aminogruppe, eine Pyrrolidino-, Piperidino-, Morpholino-, Thiomorpholino-, Piperazin-1-yl- oder 5-($C_1$–$C_3$-Alkyl)-piperazin-1-yl-Gruppe steht, $R_5$ eine Phenyl-, Chlorphenyl-, Methylphenyl- oder Methoxyphenylgruppe bedeutet und $R_6$ ein Wasserstoffatom, ein Bromatom oder einen Methylrest wiedergibt.

3. Derivate der allgemeinen Formel (I) gemäss Anspruch 1 sowie deren Salze, dadurch gekennzeichnet, dass in der Formel (I) $R_3$ ein Wasserstoffatom, eine Methylgruppe, eine Trifluormethylgruppe, eine Hydroxygruppe, einen Ethoxyrest, einen Methylthio- oder Ethylthiorest, eine Aminogruppe, eine Piperidinogruppe oder eine 4-(Methyl)-piperazin-1-yl-Gruppe bedeutet, $R_5$ für eine Phenyl-, o-Chlorphenyl-, m-Methylphenyl- oder m-Methoxyphenylgruppe steht und $R_6$ ein Wasserstoffatom, ein Bromatom oder einen Methylrest wiedergibt.

4. Derivate der Formel (I) gemäss Anspruch 1 mit den folgenden Bezeichnungen:
1,5-Diphenyl-1H-1,2,4-thiadiazin-1-oxid,
1,5-Diphenyl-6-methyl-1H-1,2,4-thiadiazin-1-oxid,
1,5-Diphenyl-3-methyl-1H-1,2,4-thiadiazin-1-oxid,
6-Brom-1,5-diphenyl-1H-1,2,4-thiadiazin-1-oxid,
1,5-Diphenyl-3-methoxy-1H-1,2,4-thiadiazin-1-oxid,
(–)-1,5-Diphenyl-3-methoxy-1H-1,2,4-thiadiazin-1-oxid,
(+)-3-Methoxy-1,5-diphenyl-1H-1,2,4-thiadiazin-1-oxid
sowie deren Additionssalze mit Säuren.

5. Verfahren zur Herstellung der neuen 1,2,4-Thiadiazine der Formel (I) gemäss Anspruch 1, worin $R_3$ und $R_5$ die in Anspruch 1 angegebene Bedeutung besitzen und $R_6$ für ein Wasserstoffatom oder einen Alkylrest mit 1 bis 3 Kohlenstoffatomen steht, sowie von deren Salzen, dadurch gekennzeichnet, dass man ein Produkt der Formel (II)

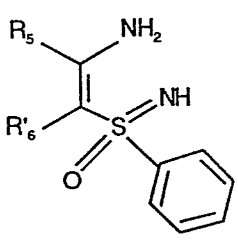

(II)

worin $R_5$ die angegebene Bedeutung besitzt und $R'_6$ ein Wasserstoffatom oder einen Alkylrest mit 1 bis 3 Kohlenstoffatomen wiedergibt, entweder mit einem Produkt der Formel (III)

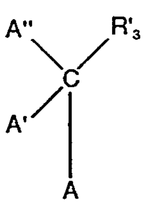

worin A, A′ und A″, die gleich oder voneinander verschieden sind, einen Ethoxyrest oder einen Dimethylaminorest darstellen und worin $R'_3$ für ein Wasserstoffatom oder eine Alkylgruppe mit 1 bis 6 Kohlenstoffatomen steht, umsetzt, um ein Produkt der Formel ($I_A$)

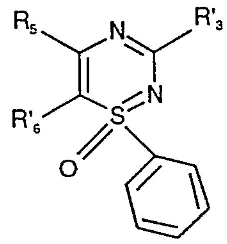

($I_A$)

zu erhalten, worin $R'_3$, $R_5$ und $R'_6$ die angegebene Bedeutung besitzen, welches man isoliert und gegebenenfalls in ein Salz überführt, oder mit einem Säurechlorid oder -anhydrid der Formel $R''_3COCl$ oder $(R''_3CO)_2O$ umsetzt, worin $R''_3$ für einen Alkylrest mit 1 bis 6 Kohlenstoffatomen, einen Cycloalkylrest mit 3 bis 6 Kohlenstoffatomen, der gegebenenfalls durch einen Alkyl- oder einen Trifluormethylrest substituiert ist, steht, um ein Produkt der Formel ($I_B$)

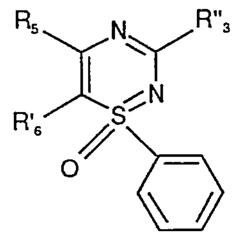

($I_B$)

zu erhalten, worin $R''_3$, $R_5$ und $R'_6$ die angegebene Bedeutung besitzen, welches man isoliert und gegebenenfalls in ein Salz überführt, oder das Produkt der Formel (II) mit N,N′-Carbonyldiimidazol der Formel ($III_A$)

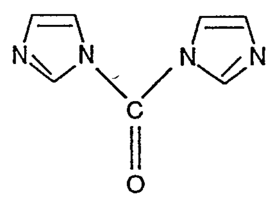

($III_A$)

umsetzt, um ein Produkt der Formel (IV)

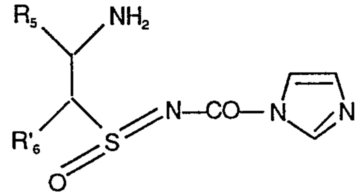

(IV)

zu erhalten, worin $R_5$ und $R'_6$ die angegebene Bedeutung besitzen, welches man gegebenenfalls

isoliert, hiernach durch Erhitzen cyclisiert, um ein Produkt der Formel ($I_C$)

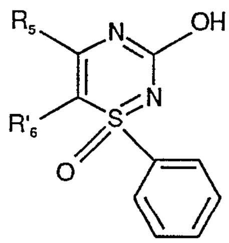

($I_C$)

zu erhalten, worin $R_5$ und $R'_6$ die angegebene Bedeutung besitzen, welches man gegebenenfalls entweder isoliert und gegebenenfalls das so erhaltene Produkt der Formel ($I_C$) in ein Salz überführt oder dieses letztere mit einem Halogenierungsmittel umsetzt, um ein Produkt der Formel (V)

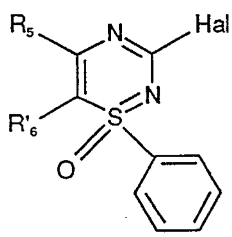

(V)

zu erhalten, worin Hal für ein Halogenatom steht und $R_5$ und $R'_6$ die angegebene Bedeutung besitzen, hiernach dieses letzere mit einem Amin der Formel

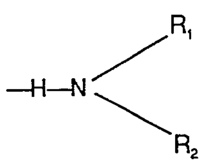

worin $R_1$ und $R_2$ wie in Anspruch 1 definiert sind, oder einem Alkohol der Formel Alc-OH oder einem Alkylthiol der Formel Alc-SH, worin Alc für einen Alkylrest mit 1 bis 6 Kohlenstoffatomen steht, umsetzt, um ein Produkt der Formel ($I_D$)

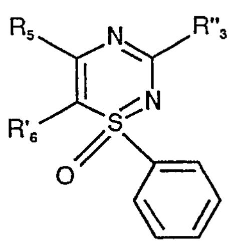

($I_D$)

zu erhalten, worin $R''_3$ für eine Gruppe

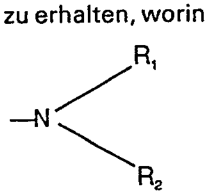

steht, in der $R_1$ und $R_2$ die angegebene Bedeutung besitzen, oder $R''_3$ eine Alkoxy- oder Alkylthiogruppe mit 1 bis 6 Kohlenstoffatomen darstellt und $R_5$ und $R'_6$ die angegebene Bedeutung besitzen, und dass man schliesslich das so erhaltene Produkt der Formel (I) isoliert und gewünschtenfalls in ein Salz überführt.

6. Verfahren gemäss Anspruch 5, dadurch gekennzeichnet, dass

(a) die Umsetzung des Produkts der Formel (II) mit dem Produkt der Formel (III) in dem Medium eines organischen Lösungsmittels, wie Dimethylformamid, bei einer Temperatur zwischen Raumtemperatur und Siedetemperatur des Reaktionsgemisches durchgeführt wird;

(b) die Umsetzung des Produkts der Formel (II) mit dem Trifluoressigsäureanhydrid in Anwesenheit einer Base, wie Triethylamin, in dem Medium eines Lösungsmittels, wie Methylenchlorid, erfolgt;

(c) die Umsetzung des Produkts der Formel (II) mit dem Produkt der Formel ($III_A$) in dem Medium eines organischen Lösungsmittels, wie Methylenchlorid, erfolgt;

(d) die Cyclisierung des Produkts der Formel (IV) durch Erhitzen des Reaktionsgemisches, vorzugsweise unter Sieden des Reaktionsgemisches, durchgeführt wird;

(e) die Halogenierung des Produkts der Formel ($I_C$) mit Hilfe eines Bromierungs- oder Chlorierungsmittels, wie Phosphoroxychlorid, erfolgt;

(f) die Umsetzung des Produkts der Formel (V) mit dem Amin der Formel

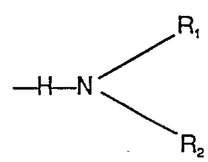

in dem Medium eines Alkohols mit niedrigem Molekulargewicht, wie Ethanol, durchgeführt wird;

(g) die Umsetzung des Produkts der Formel (V) mit dem Alkohol der Formel Alc-OH oder mit dem Thiol der Formel Alc-SH in Anwesenheit einer Base erfolgt, die im Fall des Alkohols ein Alkalicarbonat, wie Natriumcarbonat, und im Fall des Thiols ein Alkalihydrid, wie Natriumhydrid, ist, wobei die Umsetzung dann in dem Medium eines Lösungsmittels, wie Dimethylformamid oder Tetrahydrofuran, durchgeführt wird.

7. Verfahren zur Herstellung der neuen 1,2,4-Thiadiazine der Formel (I) gemäss Anspruch 1, worin $R_3$ ein Wasserstoffatom, eine Hydroxygruppe, eine Alkyl- oder Alkoxygruppe mit 1 bis 6 Kohlenstoffatomen, einen Cycloalkylrest mit 3 bis 6 Kohlenstoffatomen, der gegebenenfalls durch einen Alkylrest mit 1 bis 6 Kohlenstoffatomen substituiert ist, bedeutet, $R_5$ die in Anspruch 1 angegebene Bedeutung besitzt und $R_6$ für ein Halogenatom steht, dadurch gekennzeichnet, dass man ein Produkt der Formel ($I'_A$)

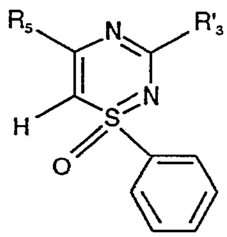

($I'_A$)

worin $R'_3$ und $R_5$ die angegebene Bedeutung besitzen, halogeniert, um ein Produkt der Formel ($I''_A$)

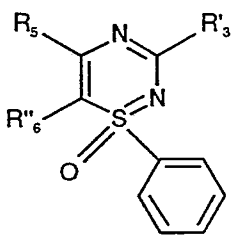

($I''_A$)

zu erhalten, in der $R'_3$ und $R_5$ die angegebene Bedeutung besitzen und $R''_6$ für ein Halogenatom steht.

8. Verfahren gemäss Anspruch 7, dadurch gekennzeichnet, dass man die Halogenierung durch Umsetzung des in Betracht gezogenen Halogens in dem Medium eines organischen Lösungsmittels, wie Chloroform, durchführt.

9. Abänderung des Verfahrens zur Herstellung der Produkte der Formel (I), worin $R_3$ einen Rest $-NH_2$ bedeutet, sowie von deren Salzen, dadurch gekennzeichnet, dass man ein Produkt der Formel (II)

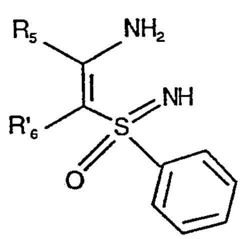

(II)

worin $R_5$ und $R'_6$ wie in Anspruch 5 definiert sind, mit Bromcyan in Gegenwart eines Reduktionsmittels umsetzt, um zu einem Produkt der Formel ($I_E$)

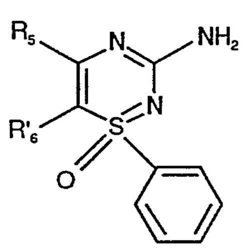

($I_E$)

zu gelangen, in der $R_5$ und $R'_6$ die angegebene Bedeutung besitzen, welches man isoliert und gegebenenfalls in ein Salz überführt.

10. Verfahren gemäss Anspruch 9, dadurch gekennzeichnet, dass das verwendete Reduktionsmittel Natriumhydrid ist und die Reduktion in dem Medium eines organischen Lösungsmittels, wie Tetrahydrofuran, durchgeführt wird.

11. Verfahren gemäss einem der Ansprüche 5, 7 oder 9, dadurch gekennzeichnet, dass man entweder von einem optisch aktiven Produkt ausgeht oder die in racemischer Form erhaltenen Produkte der Formel (I) auftrennt und die Produkte der Formel (I) in Form ihrer Enantiomeren erhält.

12. Arzneimittel, dadurch gekennzeichnet, dass sie aus den neuen 1,2,4-Thiadiazinen der Formel (I) gemäss Anspruch 1 sowie aus deren Additionssalzen mit pharmazeutisch verträglichen Säuren bestehen.

13. Arzneimittel gemäss Anspruch 12, dadurch gekennzeichnet, dass sie aus den gemäss den Ansprüchen 2 oder 3 definierten 1,2,4-Thiadiazinen sowie aus deren Additionssalzen mit pharmazeutisch verträglichen Säuren bestehen.

14. Arzneimittel gemäss Anspruch 12, dadurch gekennzeichnet, dass sie aus den Derivaten gemäss Anspruch 4 sowie aus deren Additionssalzen mit pharmazeutisch verträglichen Säuren bestehen.

15. Pharmazeutische Zusammensetzungen, dadurch gekennzeichnet, dass sie als Wirkstoff zumindest eines der Arzneimittel gemäss einem der Ansprüche 12, 13 oder 14 enthalten.

16. Als neue, industrielle Produkte die Produkte der Formel (V)

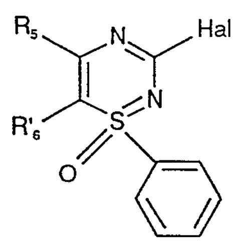

(V)

worin Hal ein Halogenatom bedeutet und $R_5$ und $R'_6$ die angegebene Bedeutung besitzen.

**Claims**

1. New 1,2,4-thiadiazines, in the form of enantiomers or mixtures of enantiomers, characterised in that they correspond to the general formula (I):

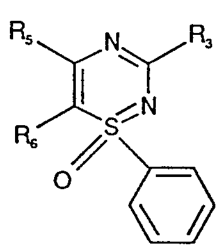

(I)

in which $R_3$ represents a hydrogen atom, an alkyl radical containing from 1 to 6 carbon atoms, a hydroxy radical, a trifluoromethyl radical, an alkoxy radical containing from 1 to 6 carbon atoms, an alkylthio radical containing from 1 to 6 carbon atoms, or $R_3$ represents an amino group, a saturated heterocycle containing from 4 to 8 carbon atoms, and able to contain another nitrogen atom, the said nitrogen atom can be substituted by an alkyl radical containing from 1 to 3 carbon atoms, $R_5$ represents a group

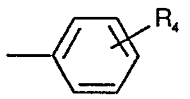

in which $R_4$ represents a hydrogen atom, a halogen atom, an alkyl radical containing from 1 to 3 carbon atoms or an alkoxy radical containing from 1 to 3 carbon atoms, $R_6$ represents an atom of hydrogen, an atom of halogen or an alkyl radical containing from 1 to 3 carbon atoms, it being understood that $R_6$ only represents a halogen atom when $R_3$ represents a hydrogen atom, a hydroxy radical, an alkyl, alkoxy or cycloalkyl radical possibly substituted by an alkyl radical as well as, if necessary, their addition salts with mineral or organic acids.

2. Derivatives corresponding to general formula (I) of the claim 1 as well as their salts, characterised in that in the said formula (I), $R_3$ represents a hydrogen atom, an alkyl radical containing from 1 to 6 carbon atoms, an alkylthio radical containing from 1 to 6 carbon atoms, a hydroxy radical, an alkoxy containing from 1 to 6 carbon atoms, a trifluormethyl radical, an amino group, a pyrrolidino, piperidino, morpholino, thiomorpholino, piperazin-1-yl or 4-(alkyl $C_{1-3}$) piperazin-1-yl radical, $R_5$ represents a phenyl, chlorophenyl, methyl-phenyl or methoxy-phenyl group, $R_6$ represents a hydrogen atom, a bromine atom or a methyl radical.

3. Derivatives corresponding to general formula (I) in claim 1 as well as their salts, characterised in that in the said formula (I), $R_3$ represents a hydrogen atom, a methyl radical, a methyl radical, a trifluoromethyl radical, a hydroxy radical, an ethoxy radical, a methylthio or ethylthio radical, an amino group, a piperidino group or a 4-(methyl)-piperazin-1-yl group, $R_5$ represents a phenyl, o-chlorophenyl, m-methyl-phenyl group or methoxy-phenyl, $R_6$ represents a hydrogen atom, a bromine atom or a methyl radical.

4. Any one of the derivatives of formula (I) as defined in claim 1, having the following names:

- 1,5-diphenyl-1H-1,2,4-thiadiazine-1-oxide,
- 1,5-diphenyl 6-methyl-1H-1,2,4-thiadiazine-1-oxide,
- 1,5-diphenyl 3-methyl-1H-1,2,4-thiadiazine-1-oxide,
- 6-bromo 1,5-diphenyl-1H-1,2,4-thiadiazine-1-oxide,
- 1,5-diphenyl 3-methoxy-1H-1,2,4-thiadiazine-1-oxide,
- (−) 1,5-diphenyl 3-methoxy-1H-1,2,4-thiadiazine-1-oxide,
- (+) 3-methoxy 1,5-diphenyl-1H-1,2,4-thiadiazine-1-oxide as well as their addition salts with acids.

5. Method of preparation of the new 1,2,4-thiadiazines as defined in formula (I) of claim 1 in which $R_3$ and $R_5$ have the meaning already given in claim 1 and $R_6$ represents a hydrogen atom or an alkyl radical containing from 1 to 3 carbon atoms, as well as their salts, characterised in that a product of formula (II):

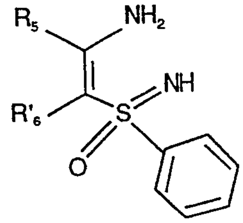

(II)

in which $R_5$ has the meaning already given and $R'_6$ represents a hydrogen atom or an alkyl radical containing from 1 to 3 carbon atoms, is made to react,

- either with a product of formula (III):

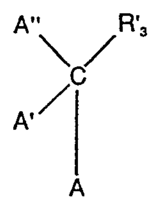

(III)

in which A, A' and A", identical or different, represent an ethoxy radical or a dimethylamino radical in which $R'_3$ represents a hydrogen atom or an alkyl radical containing from 1 to 6 carbon atoms, to obtain a product of formula ($I_A$):

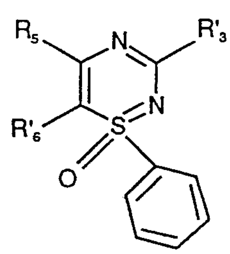

($I_A$)

in which $R'_3$, $R_5$ and $R'_6$ have the meaning already given, which is isolated and, if necessary, is salified,

- or with a chloride or acid anhydride of the formula $R''_3COCl$ or $(R''_3CO)_2O$ in which $R''_3$ represents an alkyl radical containing from 1 to 6 carbon atoms, a cycloalkyl radical containing from 3 to 6 carbon atoms possibly substituted by an alkyl radical or a trifluoromethyl radical, to obtain a product of the formula ($I_B$):

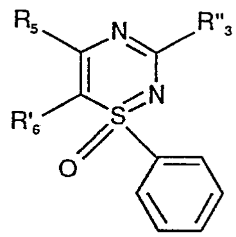

($I_B$)

in which $R''_3$, $R_5$ and $R'_6$ have the meaning already given, which is isolated, and if necessary salified.
- or the product of formula (II) is reacted with N,N'-carbonyldiimidazole of the formula ($III_A$):

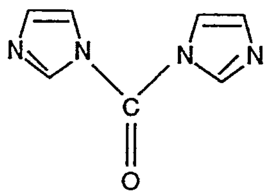

($III_A$)

to obtain a product of formula (IV)

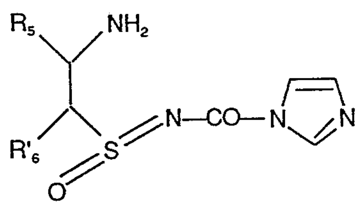

(IV)

in which $R_5$ and $R'_6$ have the meaning already given, which if necessary is isolated then cyclized by heating to obtain a product of the formula ($I_C$):

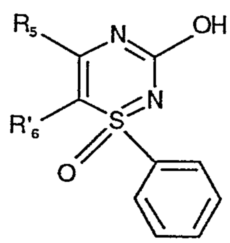

($I_C$)

in which $R_5$ and $R'_6$ have the meaning already given and that if necessary either the product with the formula ($I_C$) so obtained is isolated and if necessary salified, or this product is made to react with a hlaogenation agent, to obtain a product of formula (V):

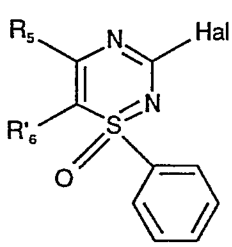

(V)

in which Hal represents a halogen atom, $R_5$ and $R'_6$ having the meaning already given, then the latter is made to react with an amine of the formula

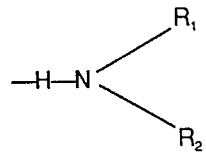

in which $R_1$ and $R_2$ have the meaning given in claim 1 or an alcohol of the formula Alk-OH or an alkylthiol of the formula Alk-SH in which Alk represents an alkyl radical containing from 1 to 6 carbon atoms, to obtain a product of the formula ($I_D$):

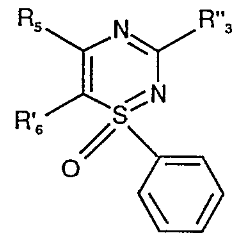

($I_D$)

in which $R''_3$ represents a

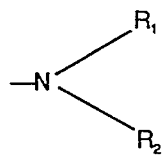

group in which $R_1$ and $R_2$ have the meaning already given or $R''_3$ represents an alkoxy or alkylthio radical containing from 1 to 6 carbon atoms, $R_5$ and $R'_6$ having the meaning already given and that finally, the product formula (I) is isolated and if desired is salified.

6. Process according to claim 5, characterised in that:

a) the reaction of the product of the formula (II) with the product of formula (III) is carried out in an organic solvent such as dimethylforamide at a temperature between ambient temperature and the boiling point of the reactive mixture.

b) the reaction of the product of formula (II) with trifluoroacetic anhydride is carried out in the presence of a base such as triethylamine, in a solvent such as dichloromethane;

c) the reaction of the product of formula (II) with the product of formula ($III_A$) is carried out in an organic solvent such as dichlormethane;

d) the cyclisation of the product of the formula (IV) is carried out by heating of the reactive mixture, preferably, at the boiling point of the reactive mixture;

e) the halogenation of the product of the formula ($I_C$) is carried out by means of a bromation or chloration agent such as phosphorus oxychloride;

f) the reaction of the product of formula (V)

with the amine of the formula $H-NR_1R_2$ is carried out in an alcohol of low molecular weight such as ethanol;

g) the reaction of the product of the formula (V) with the alcohol of the formula Alk-OH or with the thiol of the formula Alk-SH is carried out in the presence ofa base which, in the case of alcohol is an alkaline carbonate such as sodium carbonate and in the case of thiol is an alkaline hydride such as sodium hydride, the reaction being then carried out in a solvent such as dimethylformamide or tetrahydrofurane.

7. Process of preparation of the new 1,2,4-thiadiazines as defined by the formula (I) of claim 1, in which $R_3$ represents a hydrogen atom, a hydroxy radical, an alkyl or alkoxy radical containing from 1 to 6 carbon atoms, a cycloalkyl radical containing from 3 to 6 carbon atoms, possibly substituted by an alkyl radical containing from 1 to 6 carbon atoms, $R_5$ has the meaning given in claim 1 and $R_6$ represents a halogen atom, characterised in that a product of the formula ($I'_A$), in which $R'_3$ and $R_5$ have the meanings already given:

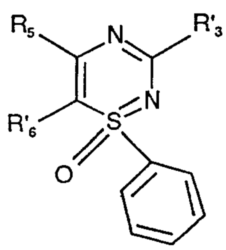

($I_A$)

is halogenated to obtain a product of the formula ($I''_A$):

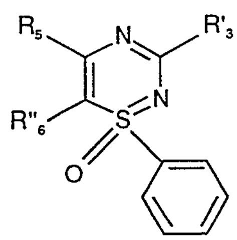

($I''_A$)

in which $R'_3$, $R_5$ have the meaning already given and $R''_6$ represents a halogen atom.

8. Process of preparation according to claim 7, characterised in that the halogenation is carried out by reaction of the halogen considered, in an organic solvent such as chloroform.

9. Variants of the process of preparation of the products with formula (I) in which $R_3$ represents an $-NH_2$ radical as well as their salts, characterised in that a product of the formula (II):

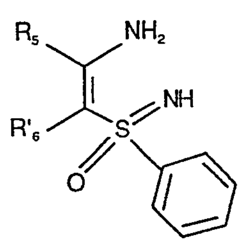

(II)

in which $R_5$ und $R'_6$ have the meanings already given in claims 5, is reacted with cyanogen bromide in the presence of a reducing agent, to obtain the product of the formula ($I_E$):

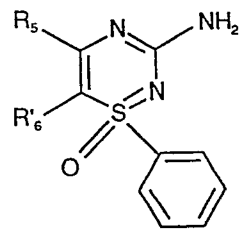

($I_E$)

in which $R_5$ and $R'_6$ have the meaning already given, which is isolated and if necessary salified.

10. Process of preparation according to claim 9, characterised in that the reducing agent used is sodium hydride and the reduction is carried out in an organic solvent such as tetrahydrofurane.

11. Process according to any one of the claims 5, 7 or 9, characterised in that either an optically active product is used at the beginning, or the products of formula (I) obtained in racemic form, are resolved and the products are obtained in their enantiomer form.

12. Medicaments, characterised in that they are constituted by the 1,2,4-thiadiazines as defined by formula (I) of claim 1 as well as their addition salts with pharmaceutically acceptable acids.

13. Medicaments, according to claim 12, characterised in that they are constituted by the 1,2,4-thiadiazines as defined in claims 2 and 3 as well as by their addition salts with pharmaceutically acceptable acids.

14. Medicaments, according to claim 12 characterised in that they are constituted by their derivatives as defined by claim 4 as well as by their addition salts with pharmaceutically acceptable acids.

15. Pharmaceutical compositions, characterised in that they contain as active principle, at least one of the medicaments as defined in the claims 12, 13 or 14.

16) As new industrial products, the products of formula (V):

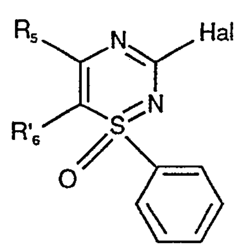

(V)

in which Hal represents a halogen atom, $R_5$ and $R'_6$ have the meanings already indicated.